# EUROPÄISCHE PATENTSCHRIFT

(11) **EP 1 485 369 B1**
(45) Veröffentlichungstag und Bekanntmachung des Hinweises auf die Patenterteilung: **20.08.2008**
(21) Anmeldenummer: 03744818.0
(22) Anmeldetag: 19.03.2003
(51) Int. Cl.: C07D 311/92, C09K 9/00

(54) **BLAUE 3H-NAPHTHO 2,1-B -PYRAN-DERIVATE SOWIE DEREN VERWENDUNG**
BLUE 3H-NAPHTHO- 2,1-B -PYRAN DERIVATIVES AND USE THEREOF
DERIVES BLEUS DE 3H-NAPHTHO 2,1-B -PYRANNE ET UTILISATION DE CES DERIVES

(30) Priorität: 21.03.2002 DE 10212717; 22.03.2002 DE 10212991
(43) Veröffentlichungstag der Anmeldung: 15.12.2004
(73) Patentinhaber: Rodenstock GmbH, 80469 München (DE)
(72) Erfinder: MANN, Claudia, 80634 München (DE); MELZIG, Manfred, 82234 Wessling (DE); WEIGAND, Udo, 80638 München (DE)
(74) Vertreter: Müller-Boré & Partner Patentanwälte
(86) Internationale Anmeldenummer: PCT/EP2003/002870
(87) Internationale Veröffentlichungsnummer: WO 2003/080595

(56) Entgegenhaltungen:
- US-A- 4 818 096
- US-A- 5 990 305
- DEMADRILLE R ET AL: "1H and 13C NMR chemical shift assignment of some 3H-naphtho[2,1-b]pyrans" MAGNETIC RESONANCE CHEMISTRY, Bd. 37, 1999, Seiten 328-330, XP009012956 USA

## Beschreibung

Die vorliegende Erfindung betrifft spezifische photochrome 3H-Naphtho[2,1-b]-pyran-Derivate sowie deren Verwendung in Kunststoffen aller Art, insbesondere für ophthalmische Zwecke. Insbesondere betrifft die vorliegende Erfindung von 3H-Naphtho[2,1-b]-pyranen abgeleitete photochrome Verbindungen, die in der offenen Form besonders langwellige Absorptionsmaxima aufweisen, wodurch bei Anwendung in phototropen Gläsern im wesentlichen blaue Farbtöne erreicht werden können, und die gleichzeitig eine hohe Eindunkelungsleistung aufweisen.

Es sind verschiedene Farbstoffklassen bekannt, die bei Bestrahlung mit Licht bestimmter Wellenlängen, insbesondere Sonnenstrahlen, reversibel ihre Farbe wechseln. Dies rührt daher, daß diese Farbstoffmoleküle durch Energiezufuhr in Form von Licht in einen angeregten farbigen Zustand übergehen, den sie bei Unterbrechung der Energiezufuhr wieder verlassen, wodurch sie in ihren farblosen oder zumindest kaum gefärbten Normalzustand zurückkehren. Zu diesen photochromen Farbstoffen gehören beispielsweise die Naphthopyrane, die im Stand der Technik mit verschiedenen Substituenten bereits beschrieben wurden.

Pyrane, speziell Naphthopyrane und von diesen abgeleitete größere Ringsysteme, sind photochrome Verbindungen, die bis heute Gegenstand intensiver Untersuchungen sind. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen.

Die im Stand der Technik bekannten Farbstoffe weisen dabei zum einen häufig eine nicht ausreichend langweilige Absorption im angeregten wie im nicht angeregten Zustand auf. Dies führt auch bei Kombinationen mit weiteren photochromen Farbstoffen zu Problemen. Zum anderen liegt häufig auch eine zu hohe Temperaturempfindlichkeit bezüglich der Eindunkelung vor, wobei gleichzeitig eine zu langsame Aufhellung eintritt. Darüberhinaus weisen die im Stand der Technik verfügbaren Farbstoffe häufig eine ungenügende Lebensdauer auf. Infolgedessen zeigen derartige Sonnenschutzgläser eine unzureichende Haltbarkeit. Letzteres macht sich in schnell nachlassender Leistung und/oder starker Vergilbung bemerkbar.

Von 2-Naphtholen abgeleitete 3H-Naphthopyrane und ihre davon durch Annellierung abgeleiteten höheren analogen Derivate sind eine Gruppe von photochromen Farbstoffen, deren längstwelliges Absorptionsmaximum der angeregten Form vorwiegend im Spektralbereich von 420 nm bis 500 nm liegt und somit einen gelben, orangen oder rotorangen Farbeindruck vermitteln (siehe US-A-5,869,658 sowie US-A-6,022,495). Für neutral eindunkelnde phototrope Gläser werden jedoch leistungsstarke violette bis blaue photochrome Farbstoffe benötigt. Violette bis blaue photochrome Farbstoffe, die derzeit im Stand der Technik verfügbar sind, stammen üblicherweise aus der Klasse der Spiroxazine, Fulgide oder 2H-Naphtho[1,2-b]pyrane. Spiroxazin-Farbstoffe sind jedoch in der Regel hinsichtlich der Hochtemperaturleistung nachteilig, während Fulgid-Farbstoffe in der Lebensdauer und 2H-Naphtho[1,2-b]pyrane in der Aufhellgeschwindigkeit keine für die Anwendung in Sonnenschutzgläser vollständig zufriedenstellenden Eigenschaften aufweisen.

Die Einführung von elektronenschiebenden Substituenten an den Arylresten in o-Stellung zum Pyransauerstoff, wie beispielsweise in WO 98/45281, WO 01/12619 und EP 0 945 451 A1 beschrieben, führt zu rot bzw. rotviolett eindunkeinden 3H-Naphtho[2,1-b]pyranen. In WO 01/12619 werden Verbindungen offenbart, deren eine geminale Arylgruppe eine para-aminosubstituierte Gruppe und deren andere Arylgruppe eine in meta- oder para-Stellung substituierte Alkoxy- oder Thioalkoxygruppe aufweisen, wobei dieses Substitutionsmuster einen positiven Einfluß auf die Aufhellgeschwindigkeit bewirkt. In WO 98/45281 werden rote hyperchrome Verbindungen beschrieben, die zusätzlich eine Aminfunktion vorrangig in 6-Stellung der 3H-Naphtho[2,1-b]pyran-Einheit enthalten. Verbindungen mit nicht ausgeprägten basischen Aminogruppen werden in EP 0 945 451 A1 beschrieben, die im angeregten Zustand eine pinkfarbene bis violette Farbe zeigen sowie eine ansprechende Lebensdauerleistung aufweisen. 3H-Naphtho[2,1-b]pyrane mit Arylsubstituenten in der 6-Stellung sind außerdem in WO 99/31082 beschrieben. Die Auswirkung der Arylsubstitution in der 6-Stellung auf das längstwellige Absorptionsmaximum der nicht angeregten sowie der angeregten Form ist bei diesen Verbindungen jedoch sehr gering.

Entsprechende Substitution in der 8-Stellung der 3H-Naphtho[2,1-b]pyran-Einheit bewirkt eine bathochrome Verschiebung des längstwelligen Absorptionsmaximums, vor allem durch die Einführung von Alkoxygruppen, wie in US-A-5,238,981 beschrieben. Außerdem sind auch Verbindungen mit Dialkylaminogruppen in 8-Stellung offenbart. Die Verwendung von Stickstoff-Heterocyclen als Substituenten in der 8-Stellung der 3H-Naphtho[2,1-b]pyran-Einheit wird in US-A-5,990,305 erwähnt, wodurch im Gegensatz zu offenkettigen Aminogruppen eine verbesserte Lebensdauer erzielt wird. Diese wird auch mit Substituenten erreicht, die sog. HALS-(hindered amine light stabilizer) Struktureinheiten enthalten. Schließlich sind in DE 102 00 040 violette bis blaue 3H-Naphtho[2,1-b]pyrane beschrieben, die Aminogruppen-enthaltende Substituenten sowohl in 3-Stellung als auch 8-Stellung aufweisen.

Magnetic Resonance Chemistry, Bd. 37, 1999, Seiten 328-330, beschreibt lediglich 8-(N-Methylpiperazin)-3,3-diphenyl-3H-naphtho[2,1-b]-pyran.

US-A-4,818,096 beschreibt blaue 3H-Naphtho[2,1-b]-pyrane, die in der 3-Stellung mit einer Aminogruppe oder Stickstoff-Heterocyclen substituiert sind.

Somit liegt der vorliegenden Erfindung die Aufgabe zugrunde, neue photochrome Farbstoffe bereitzustellen, die verbesserte Eigenschaften gegenüber den im Stand der Technik beschriebenen Verbindungen aufweisen sollen. Die photochromen Verbindungen sollen sich gegenüber vergleichbaren Verbindungen aus dem Stand der Technik insbesondere durch eine im angeregten Zustand verbesserte Kombination von langwelliger Absorption und hoher Eindunklungsleistung bei gleichzeitig guten Kinetik- und Lebensdauereigenschaften, d.h. schnelle Aufhellungsgeschwindigkeit sowie gutes Verhalten im Lebensdauertest, auszeichnen.

Diese Aufgabe wird durch die in den Ansprüchen gekennzeichneten Gegenstände gelöst.

Insbesondere werden photochrome 3H-Naphtho[2,1-b]-pyran-Derivate mit der allgemeinen Formel (I) bereitgestellt: 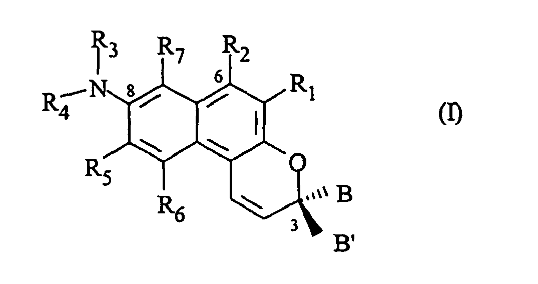 worin
die Reste R₁, R₅, R₆ und R₇ unabhängig voneinander Wasserstoff oder einen Substituenten darstellen, ausgewählt aus der Gruppe α, bestehend aus Fluor, Chlor, Brom, Hydroxy, Silyloxy, Amino, einem geradkettigen oder verzweigtkettigen (C₁-C₆)-Alkylrest, einem (C₃-C₇)-Cycloalkylrest, einem geradkettigen oder verzweigtkettigen (C₁-C₆)-Alkoxyrest, Morpholinyl, Thiomorpholinyl, Piperidinyl, Piperazinyl, Pyrrolidinyl, Imidazolinyl, Pyrazolidinyl, 1,2,3,4-Tetrahydrochinolinyl, 1,2,3,4-Tetrahydroisochinolinyl, Perhydroazepinyl, 4-Methyl-perhydro-1,4-diazepinyl, Perhydroazocinyl, Phenyl, Phenoxy, Benzyl, Benzyloxy, Naphthyl, Naphthoxy, Phenanthryl oder Pyridyl, die jeweils mit ein, zwei oder drei Substituenten, unabhängig voneinander ausgewählt aus der Gruppe β, bestehend aus einem geradkettigen oder verzweigtkettigen (C₁-C₆)-Alkylrest, einem (C₃-C₇)-Cycloalkylrest, einem geradkettigen oder verzweigtkettigen (C₁-C₆)-Alkoxyrest, Hydroxy, tert.-Butyldiphenylsilyloxy, Amino, Di(C₁-C₆)alkylamino, Nitro, Cyan, Benzyl, 4-Methoxybenzyl, 4-Nitrobenzyl, Phenyl, 4-Methoxyphenyl, 4-Dimethylaminophenyl, Pyridyl und Pyrimidinyl, substituiert sein können;
der Rest R₂ aus phenyl, Naphthyl, phenanthryl ,pyridyl, Chinolinyl, Isochinolinyl, Thienyl, Benzothienyl, Dibenzothienyl, Furanyl, Benzofuranyl, Dibenzofuranyl, Carbazolyl, Phenothiazinyl, Phenoxazinyl, Oxazolyl, Benzoxazolyl, Oxadiazolyl, Thiazolyl, Benzothiazolyl, Thiadiazolyl, Imidazolyl, Pyrazolyl, Triazolyl, Tetrazolyl, Pyrimidinyl, Pyrazinyl, Acetyl, Benzoyl, Cyan, Formyl, Iminomethyl, 1-Imino-aminomethyl, N-Hydroxyiminomethyl, Methylenamino, Cyanamino, Cyanmethyl, Dicyanmethyl, Carboxy, Carboxymethyl, (C₁-C₆)-Acyloxy, (C₁-C₆)-Alkoxycarbonyl, (C₁-C₆)-Alkoxycarbonyl-methyl, Phenoxycarbonyl, Benzyloxycarbonyl, Nitro, Diazophenyl, Aminocarbonyl, Ethenyl, 4-Ethenylphenyl, Ethinyl oder 4-Ethinylphenyl oder unter Ausbildung einer kationischen Struktur aus N-(C₁-C₆)-Alkylpyridinio, 1-Pyridinio, N-(C₁-C₆)-Alkylchinolinio, 1-Chinolinio, N-(C₁-C₆)-Alkylisochinolinio, 2-lsochinolinio, Iminiomethyl oder 1-lminioaminomethyl, wobei dann das entsprechende Gegenion aus Chlorid, Bromid, Sulfat, Hydrogensulfat, Tetrafluoroborat, Hexafluorophosphat, Mesylat, Tosylat oder Triflat ausgewählt ist, wobei die vorstehenden Substituenten, soweit möglich, jeweils ein, zwei, drei oder vier Substituenten aus der Gruppe β aufweisen können, ausgewählt ist,
oder der Rest R₂ für eine -Y-Naphthyl-Gruppe steht, wobei Y dabei ein Linker zwischen dem Naphthylrest und der Naphthopyraneinheit ist, ausgewählt aus einer Einfachbindung (direkte Verbindung) oder aus CH₂, Ethandiyl, Ethendiyl, Ethindiyl, Iminomethyl, Phenylen, Biphenyldiyl, Ferrocendiyl, Pyridindiyl, Pyrimidindiyl, Pyrazindiyl oder (Bipyrimidinyl)diyl, und wobei der Naphthylrest einen, zwei, drei oder vier Substituenten aufweisen kann, ausgewählt aus der Gruppe α oder einer -NR₃R₄ Gruppe, wie nachstehend definiert, wobei einer der Naphthylsubstituenten zusammen mit dem Rest R₄ der an den Naphthylrest gebundenen -NR₃R₄ Gruppe eine an den aromatischen Ring des Naphthylrests gebundene -R₄N-(CH₂)ₖ-X-Gruppe mit k = 1 oder 2 bilden kann, wobei der Rest R₄ dann Wasserstoff, einen geradkettigen oder verzweigtkettigen (C₁-C₆)-Alkylrest oder Phenyl darstellt und X Sauerstoff, Schwefel, CH₂, C(CH₃)₂, C(C₆H₅)₂, NCH₃ oder NPh darstellt, und wobei an diese -R₄N-(CH₂)ₖ-X-Gruppe wiederum ein Benzoring annelliert sein kann; oder wobei zwei der Naphthylsubstituenten einen an den Naphthylrest der -Y-Naphthyl-Gruppe annellierten Pyranring bilden, der wiederum mit B und B', wie nachstehend definiert, substituiert sein kann, so daß über den Linker Y gebunden ein zweites photochromes Naphthopyran-System vorliegt;
die Reste R₃ und R₄ unabhängig voneinander aus Wasserstoff, einem geradkettigen oder verzweigtkettigen (C₁-C₆)-Alkylrest, einem (C₃-C₇)-Cycloalkylrest, einem (C₁-C₆)-Alkoxyrest, Phenyl oder Benzyl, wobei die beiden letzteren einen oder mehrere Substituenten aus der Gruppe β aufweisen können, ausgewählt sind, oder
die Reste R₃ und R₄ zusammen mit dem Stickstoffatom eine Azaadamantylgruppe oder einen 3- bis 10-gliedrigen Stickstoffheterocyclus bilden, der unsubstituiert oder mit einem geradkettigen oder verzweigtkettigen (C₁-C₆)-Alkylrest substituiert sein kann, wobei der Stickstoffheterocyclus ein oder mehrere Heteroatome aus der Gruppe O, S oder NR⁸ enthalten kann, wobei der Rest R⁸ aus Wasserstoff oder der Gruppe β ausgewählt ist, und wobei der Stickstoffheterocyclus mit einem oder zwei Benzolringen annelliert sein kann,
oder die Reste R₄ und R₅ bzw. R₃ und R₇ unter Einschließen des Stickstoffatoms jeweils miteinander eine an den Benzolring des Naphthopyrangerüsts gebundene -R₄N-(CH₂)ₖ-X- bzw. -R₃N-(CH₂)ₖ-X- Einheit mit k = 1 oder 2 bilden, wobei X aus O, S, CH₂, C(CH₃)₂, C(C₆H₅)₂, N(CH₃) oder N(C₆H₅) ausgewählt ist und der Rest R₄ bzw. R₃ dann aus Methyl oder Phenyl ausgewählt ist, wobei an diese -R₄N-(CH₂)ₖ-X- bzw. -R₃N-(CH₂)ₖ-X- Einheit wiederum ein Benzolring annelliert sein kann, oder
die Reste NR₃R₄, R₅ und R₇ zusammen mit dem Benzolring des Naphthopyrangerüsts, an den sie gebunden sind, eine Julolidinyl-Einheit bilden, und
B und B' unabhängig voneinander ausgewählt sind aus un-, mono- oder disubstituiertem Phenyl, Ethinyl, Ethenyl, Naphthyl, Furanyl, Benzofuranyl, Thienyl, Benzothienyl oder Julolidinyl, wobei die Substituenten aus der Gruppe α sowie Ethenyl, 4-Ethenylphenyl, Ethinyl oder 4-Ethinylphenyl ausgewählt sind, wobei die Substituenten aus der Gruppe α sowie die vorstehenden Substituenten, soweit möglich, wiederum jeweils unabhängig voneinander zwei oder drei Substituenten aus der Gruppe β aufweisen können, oder wobei zwei direkt benachbarte Substituenten eine X-(CH₂)_{q}-Z-Gruppierung darstellen, wobei q = 1, 2 oder 3 ist und X und Z unabhängig voneinander Sauerstoff, Schwefel, NCH₃, NPh, CH₂, C(CH₃)₂ oder C(C₆H₅)₂ sein können, oder
B und B' zusammen ein un-, mono- oder disubstituiertes 9-Spirofluoren darstellen, wobei die Fluoren-Substituenten aus der Gruppe β ausgewählt sind, oder B und B' zusammen einen gesättigten Kohlenwasserstoff darstellen, der C₃-C₁₂ spiromonocyclisch, C₇-C₁₂ spirobicyclisch oder C₇-C₁₂ spirotricyclisch ist.

Durch Einführung eines Substituenten, der eine Aminogruppe aufweist, in der 8-Position am Naphthopyran-Grundgerüst und gleichzeitig eines spezifischen Substituenten in der 6-Stellung, insbesondere eines solchen, der das π-Elektronensystem des Moleküls vergrößert, lassen sich die photochromen Eigenschaften von 3H-Naphtho[2,1-b]-pyranen, inbesondere deren Eindunkelungsleistung, deutlich steigern. Gegenüber entsprechenden Verbindungen aus dem Stand der Technik werden erstmals 3H-Naphtho[2,1-b]pyrane bereitgestellt, die in der angeregten Form deutlich längerwellig absorbieren und gleichzeitig eine hohe Eindunklungsleistung aufweisen. Dadurch lassen sich blaue photochrome Farbstoffe in nur wenigen Reaktionsschritten herstellen. Darüber hinaus zeigen die erfindungsgemäßen photochromen 3H-Naphtho[2,1-b]-pyrane gute Lebensdauer und schnelle Aufhellgeschwindigkeiten bei einer außergewöhnlichen Eindunkelungsleistung.

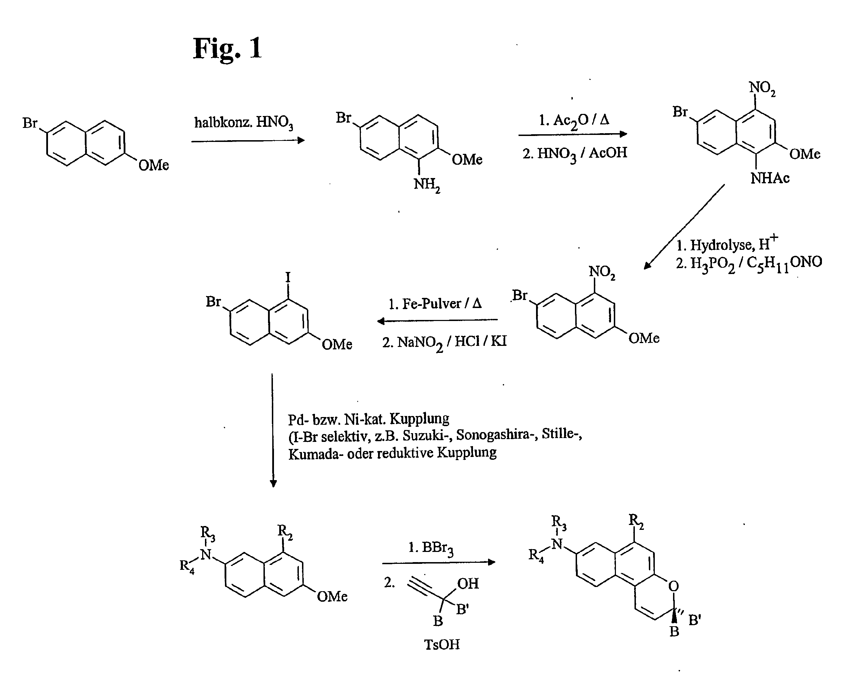

Fig. 1 zeigt exemplarisch einen Syntheseweg zur Herstellung beispielhafter erfindungsgemäßer photochromer Verbindungen.

In einer bevorzugten Ausführungsform der vorliegenden Erfindung ist der Rest R₂ aus Phenyl, Naphthyl, Pyridyl, Oxazolyl, Oxadiazolyl, Thiazolyl, Thiadiazolyl, Imidazolyl, Pyrazolyl, Triazolyl, Tetrazolyl, Pyrimidinyl, Pyrazinyl, Acetyl, Benzoyl, Cyan, Formyl, Iminomethyl, 1-Imino-aminomethyl, 1-(Aminomethylen)-2-iminoethyl, N-Hydroxyiminomethyl, Methylenamino, Cyanamino, Dicyanmethyl, Carboxymethyl, (C₁-C₆)-Alkoxycarbonyl, Nitro, Ethenyl, 4-Ethenylphenyl, Ethinyl oder 4-Ethinylphenyl, welche jeweils, soweit möglich, ein, zwei, drei oder vier Substituenten aus der Gruppe β aufweisen können, ausgewählt oder der Rest R₂ steht für eine -Y-Naphthyl-Gruppe, wobei Y dabei ein Linker zwischen dem Naphthylrest und dem Naphthopyran ist, ausgewählt aus einer Einfachbindung oder CH₂, Ethandiyl, Ethendiyl, Ethindiyl, Iminomethyl, Phenylen, Biphenyldiyl, Ferrocendiyl, Pyridindiyl, Pyrimidindiyl, Pyrazindiyl oder (Bipyrimidinyl)diyl, und wobei der Naphthylrest einen, zwei, drei oder vier Substituenten aufweisen kann, ausgewählt aus der Gruppe α oder einer -NR₃R₄-Gruppe, wie bereits vorstehend definiert, wobei einer der Naphthylsubstituenten zusammen mit dem Rest R₄ der an den Naphthylrest gebundenen -NR₃R₄ Gruppe eine an den aromatischen Ring des Naphthylrests gebundene -R₄N-(CH₂)ₖ-X-Gruppe mit k = 1 oder 2 bilden kann, wobei der Rest R₄ dann Wasserstoff, einen geradkettigen oder verzweigtkettigen (C₁-C₆)-Alkylrest oder Phenyl darstellt und X Sauerstoff, Schwefel, CH₂, C(CH₃)₂, C(C₆H₅)₂, NCH₃ oder NPh darstellt, und wobei an diese -R₄N-(CH₂)ₖ-X-Gruppe wiederum ein Benzoring annelliert sein kann; oder wobei zwei der Naphthylsubstituenten einen an den Naphthylrest der -Y-Naphthyl-Gruppe annellierten Pyranring bilden, der wiederum mit B und B', wie bereits vorstehend definiert, substituiert sein kann, so daß über den Linker Y gebunden ein zweites photochromes Naphthopyran-System vorliegt, d.h. ein gemäß der vorstehenden Formel (I) gekennzeichnetes 3H-Naphtho[2,1-b]-pyran kann über den Linker Y mit einem weiteren, gleichen oder unterschiedlichen 3H-Naphtho[2,1-b]-pyran verbunden sein.

In einer besonders bevorzugten Ausführungsform steht der Rest R₂ für Phenyl, 4-Methoxyphenyl, 4-Nitrophenyl, 4-(2-Pyrimidinyl)-phenyl, 2-Pyridyl, 3-Pyridyl, 4-Pyridyl, 2-Pyrimidinyl, 2-Pyrazinyl, 5-Methyl-2-oxadiazolyl, 2,4-Dimethyl-1-pyrazolyl, 4,5-Dimethyl-2-imidazolyl, 1-Hex-1-inyl, Phenylethinyl oder 4-Pyridylethinyl oder der Rest R₂ stellt einen Linker zwischen den 6-Substituenten zweier wie vorstehend definierter 3H-Naphtho[2,1-b]pyrane dar, wobei der Linker aus einer direkten Verbindung, Ethendiyl, Ethindiyl, 1,4-Phenylen oder 5,5'-Bipyrimidinyl-2,2'-diyl ausgewählt ist.

In einer weiteren bevorzugten Ausführungsform der vorliegenden Erfindung werden die Reste R₃ und R₄ gemäß der vorstehenden Formel (1) aus einem unsubstituierten, mono- oder disubstituierten Phenyl-, Phenoxy-, Benzyl-, Benzyloxy-, Naphthyl- oder Naphthoxyrest ausgewählt oder bilden zusammen mit dem Stickstoffatom einen 3- bis 10-gliedrigen Stickstoffheterocyclus, insbesondere eine Morpholingruppe, eine Thiomorpholingruppe, eine Piperidingruppe, eine Azacycloheptangruppe, eine Azacyclooctangruppe, eine 1,4-Diaza-1-methyl-cycloheptangruppe, eine Piperazingruppe, eine (N'-(C₁-C₆-Alkyl)piperazingruppe, eine Pyrrolidingruppe, eine lmidazolidingruppe, eine Pyrazolidingruppe, eine Aziridingruppe, eine Azetidingruppe, eine Indolingruppe, eine Carbazolgruppe, eine Phenothiazingruppe, eine Phenazingruppe, eine Phenoxazingruppe, eine Tetrahydrochinolingruppe oder eine Tetrahydroisochinolingruppe. Mehr bevorzugt steht die NR₃R₄-Gruppe in der vorstehenden Formel (I) in ihrer Gesamtheit für Diphenylamino, Dianisylamino, Morpholinyl, Thiomorpholinyl, 3,5-Dimethylthiomorpholinyl, Piperidinyl, Azacycloheptyl, Azacyclooctyl, 1,4-Diaza-1-methyl-cycloheptyl, Piperazinyl, Pyrrolidinyl oder 1,2,3,4-Tetrahydroisochinolinyl.

Wenn die Reste NR₃R₄, R₅ und R₇ zusammen mit dem Benzolring des Naphthopyrangerüsts, an den sie gebunden sind, eine Julolidinyl-Einheit bilden, ergibt sich folgende Struktureinheit:

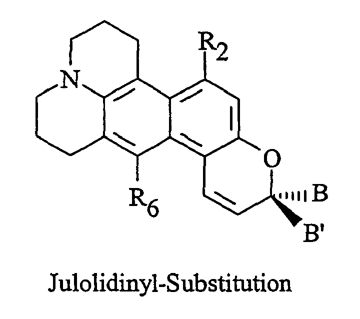

Wenn die Reste R₃ und R₇ bzw. R₄ und R₅ unter Einschließen des Stickstoffatoms jeweils miteinander eine an den Benzolring des Naphthopyrangerüsts gebundene -R₃N-(CH₂)ₖ-X- bzw. -R₄N-(CH₂)ₖ-X- Einheit, wie vorstehend definiert, bilden, so sind folgende Struktureinheiten bevorzugt:

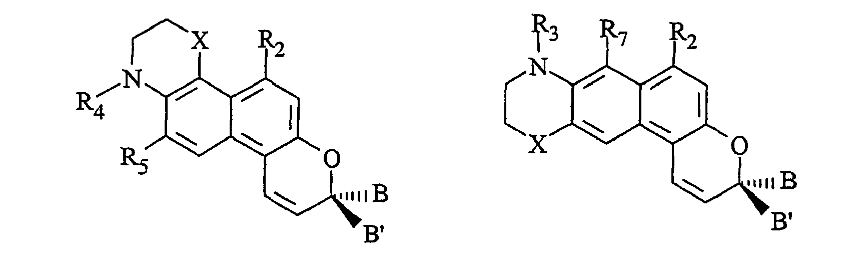

Vorzugsweise ist X in den vorstehenden Struktureinheiten aus O, CH₂ und N(CH₃) ausgewählt.

In einer weiteren bevorzugten Ausführungsform der vorliegenden Erfindung ist mindestens einer der Reste B und B' aus einem in para-Stellung mit einer wie vorstehend definierten -NR₃R₄ Gruppe substituierten Phenylrest ausgewählt. Dabei können die Reste R₃ und R₄ zusammen mit dem Stickstoffatom dieser -NR₃R₄ Gruppe auch eine Azaadamantylgruppe bilden. Alternativ können sie auch einen wie bereits vorstehend definierten 3- bis 10-gliedrigen Stickstoffheterocyclus, insbesondere eine Morpholingruppe, eine Thiomorpholingruppe, eine Piperidingruppe, eine Azacycloheptangruppe, eine Azacyclooctangruppe, eine 1,4-Diaza-1-methyl-cycloheptangruppe, eine Piperazingruppe, eine N-(N'-(C₁-C₆-Alkyl)piperazingruppe oder eine Pyrrolidingruppe bilden, oder der in para-Stellung mit einer -NR₃R₄ Gruppe substituierte Phenylrest steht im Gesamten für eine in 6-Position gebundene N-Methyl-1,2,3,4-tetrahydrochinolinylgruppe, so daß folgende Struktureinheit vorliegt:

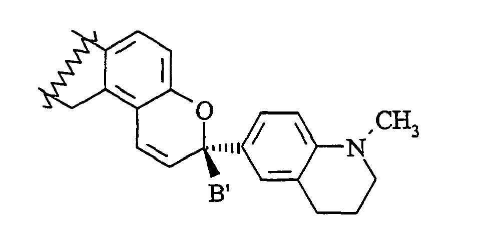

In einer anderen Ausführungsform ist mindestens einer der Reste B und B' vorzugsweise eine 4-Dimethylaminophenylgruppe.

Wenn einer der Reste B und B' für einen an den Pyranring über die 3-Position gebundenen Julolidinylrest steht, ergibt sich folgende Struktureinheit:

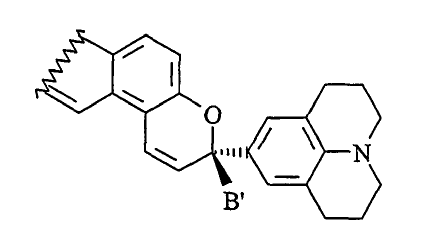

Die längstwelligen Absorptionsmaxima der offenen (farbigen) Form von zwei beispielhaften photochromen 3H-Naphtho[2,1-b]pyran-Derivaten gemäß der vorliegenden Erfindung sind in der nachstehenden Tabelle exemplarisch aufgeführt.

| Verbindung | R₂ | NR₃R₄ | B | B' | R₁=R₅=R₆ =R₇ | λₘₐₓ. |
|---|---|---|---|---|---|---|
| (1) | H | N-Piperidinyl | 4-Dimethylaminophenyl | Phenyl | H | 570 nm |
| (2) | Phenyl | N-Piperidinyl | 4-Dimethylaminophenyl | Phenyl | H | 585 nm |
| (3) | Phenyl-ethinyl | N-Piperidinyl | 4-Dimethylaminophenyl | Phenyl | H | 615 nm |

| | | | | | | |
|---|---|---|---|---|---|---|
| λₘₐₓ : Längstwelliges Absorptionsmaximum der offenen (farbigen) Form [gemessen in Methacrylatpolymer] | | | | | | |

Verbindung (1) ist eine Vergleichsverbindung gemäß dem Stand der Technik (DE 102 00 40), worin der Rest R₂ Wasserstoff ist. Ein Vergleich der Verbindung (1) mit der erfindungsgemäßen Verbindung (2), worin der Rest R₂ Phenyl ist, bzw. mit der Verbindung (3), worin der Rest R₂ Phenylethinyl ist, zeigt deutlich, dass durch Einführung eines Substituenten in der 6-Stellung 3H-Naphtho[2,1-b]pyrane bereitgestellt werden, die in der angeregten Form deutlich längerwellig absorbieren.

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen 3H-Naphtho[2,1-b]-pyran-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen photochromen Farbstoffe beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen 3H-Naphtho[2,1-b]-pyran-Farbstoffe durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfaßt beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen 3H-Naphtho[2,1-b]-pyran-Farbstoffe beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

Die erfindungsgemäßen photochromen 3H-Naphtho[2,1-b]-pyran-Farbstoffe mit der allgemeinen Formel (1) lassen sich beispielsweise nach dem in Figur 1 gezeigten Reaktionsschema herstellen.

Schlüsselschritt der Synthese der erfindungsgemäßen Verbindungen sind ausgehend von 6-Brom-4-iod-2-methoxynaphthalin Palladium- bzw. Nickel-katalysierte Kupplungen mit ausgeprägter I/Br-Selektivität. Durch die geeignete Wahl der Reaktionsbedingungen, wie sie einem Fachmann bekannt sind, ist es möglich, nur die lod-Gruppe mit dem Rest R₂ zu substituieren, z.B. mittels Kupplungen vom Suzuki-, Sonogashira-, Stille- oder Kumada-Typ oder einer reduktiven Dimerisierungs-Reaktion. Anschließend wird die (substituierte) Amino-Gruppe am Naphthalin-System mittels Palladium-katalysierter Aminierung eingeführt. Nach Etherspaltung werden die so erhaltenen 2-Naphthol-Derivate mit geeignet substituierten 2-Propin-1-ol-Derivaten zu den erfindungsgemäßen Verbindungen umgesetzt.

## Patentansprüche

1. Photochrome 3H-Naphtho[2,1-b]-pyrane mit der allgemeinen Formel (I). 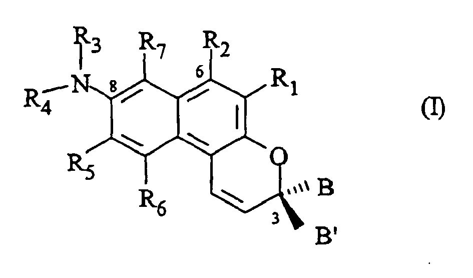 worin
die Reste R₁, R₅, R₆ und R₇ unabhängig voneinander Wasserstoff oder einen Substituenten darstellen, ausgewählt aus der Gruppe α, bestehend aus Fluor, Chlor, Brom, Hydroxy, Silyloxy, Amino, einem geradkettigen oder verzweigtkettigen (C₁-C₆)-Alkylrest, einem (C₃-C₇)-Cycloalkylrest, einem geradkettigen oder verzweigtkettigen (C₁-C₆)-Alkoxyrest, Morpholinyl, Thiomorpholinyl, Piperidinyl, Piperazinyl, Pyrrolidinyl, Imidazolinyl, Pyrazolidinyl, 1,2,3,4-Tetrahydrochinolinyl, 1,2,3,4-Tetrahydroisochinolinyl, Perhydroazepinyl, 4-Methyl-perhydro-1,4-diazepinyl, Perhydroazocinyl, Phenyl, Phenoxy, Benzyl, Benzyloxy, Naphthyl, Naphthoxy, Phenanthryl oder Pyridyl, die jeweils mit ein, zwei oder drei Substituenten, unabhängig voneinander ausgewählt aus der Gruppe β, bestehend aus einem geradkettigen oder verzweigtkettigen (C₁-C₆)-Alkylrest, einem (C₃-C₇)-Cycloalkylrest, einem geradkettigen oder verzweigtkettigen (C₁-C₆)-Alkoxyrest, Hydroxy, tert.-Butyldiphenylsilyloxy, Amino, Di(C₁-C₆)alkylamino, Nitro, Cyan, Benzyl, 4-Methoxybenzyl, 4-Nitrobenzyl, Phenyl, 4-Methoxyphenyl, 4-Dimethylaminophenyl, Pyridyl und Pyrimidinyl, substituiert sein können;
der Rest R₂ aus Phenyl Naphthyl, phenanthryl, pyridyl, Chinolinyl, Isochinolinyl, Thienyl, Benzothienyl, Dibenzothienyl, Furanyl, Benzofuranyl, Dibenzofuranyl, Carbazolyl, Phenothiazinyl, Phenoxazinyl, Oxazolyl, Benzoxazolyl, Oxadiazolyl, Thiazolyl, Benzothiazolyl, Thiadiazolyl, Imidazolyl, Pyrazolyl, Triazolyl, Tetrazolyl, Pyrimidinyl, Pyrazinyl, Acetyl, Benzoyl, Cyan, Formyl, Iminomethyl, 1-lmino-aminomethyl, N-Hydroxyiminomethyl, Methylenamino, Cyanamino, Cyanmethyl, Dicyanmethyl, Carboxy, Carboxymethyl, (C₁-C₆)-Acyloxy, (C₁-C₆)-Alkoxycarbonyl, (C1₁-C₆)-Alkoxycarbonyl-methyl, Phenoxycarbonyl, Benzyloxycarbonyl, Nitro, Diazophenyl, Aminocarbonyl, Ethenyl, 4-Ethenylphenyl, Ethinyl oder 4-Ethinylphenyl oder unter Ausbildung einer kationischen Struktur aus N-(C₁-C₆)-Alkylpyridinio, 1-Pyridinio, N-(C₁-C₆)-Alkylchinolinio, 1-Chinolinio, N-(C₁-C₆)-Alkylisochinolinio, 2-Isochinolinio, lminiomethyl oder 1-Iminioaminomethyl, wobei dann das entsprechende Gegenion aus Chlorid, Bromid, Sulfat, Hydrogensulfat, Tetrafluorborat, Hexafluorphosphat, Mesylat, Tosylat oder Triflat ausgewählt ist, wobei die vorstehenden Substituenten, soweit möglich, jeweils ein, zwei, drei oder vier Substituenten aus der Gruppe β aufweisen können, ausgewählt ist,
oder der Rest R₂ für eine -Y-Naphthyl-Gruppe steht, wobei Y dabei ein Linker zwischen dem Naphthylrest und der Naphthopyraneinheit ist, ausgewählt aus einer Einfachbindung oder aus CH₂, Ethandiyl, Ethendiyl, Ethindiyl, lminomethyl, Phenylen, Biphenyldiyl, Ferrocendiyl, Pyridindiyl, Pyrimidindiyl, Pyrazindiyl oder (Bipyrimidinyl)diyl, und wobei der Naphthylrest einen, zwei, drei oder vier Substituenten aufweisen kann, ausgewählt aus der Gruppe α oder einer -NR₃R₄-Gruppe, wie nachstehend definiert, wobei einer der Naphthylsubstituenten zusammen mit dem Rest R₄ der an den Naphthylrest gebundenen -NR₃R₄ Gruppe eine an den aromatischen Ring des Naphthylrests gebundene -R₄N-(CH₂)ₖ-X-Gruppe mit k = 1 oder 2 bilden kann, wobei der Rest R₄ dann Wasserstoff, einen geradkettigen oder verzweigtkettigen (C₁-C₆)-Alkylrest oder Phenyl darstellt und X Sauerstoff, Schwefel, CH₂, C(CH₃)₂, C(C₆H₅)₂, NCH₃ oder NPh darstellt, und wobei an diese -R₄N-(CH₂)ₖ-X-Gruppe wiederum ein Benzoring annelliert sein kann; oder wobei zwei der Naphthylsubstituenten einen an den Naphthylrest der -Y-Naphthyl-Gruppe annellierten Pyranring bilden, der wiederum mit B und B', wie nachstehend definiert, substituiert sein kann, so daß über den Linker Y gebunden ein zweites photochromes Naphthopyran-System vorliegt;
die Reste R₃ und R₄ unabhängig voneinander aus Wasserstoff, einem geradkettigen oder verzweigtkettigen (C₁-C₆)-Alkylrest, einem (C₃-C₇)-Cycloalkylrest, einem (C₁-C₆)-Alkoxyrest, Phenyl oder Benzyl, wobei die beiden letzteren einen oder mehrere Substituenten aus der Gruppe β aufweisen können, ausgewählt sind, oder
die Reste R₃ und R₄ zusammen mit dem Stickstoffatom eine Azaadamantylgruppe oder einen 3- bis 10-gliedrigen Stickstoffheterocyclus bilden, der unsubstituiert oder mit einem geradkettigen oder verzweigtkettigen (C₁-C₆)-Alkylrest substituiert sein kann, wobei der Stickstoffheterocyclus ein oder mehrere Heteroatome aus der Gruppe O, S oder NR⁸ enthalten kann, wobei der Rest R⁸ aus Wasserstoff oder der Gruppe β ausgewählt ist, und wobei der Stickstoffheterocyclus mit einem oder zwei Benzolringen annelliert sein kann, oder
die Reste R₄ und R₅ bzw. R₃ und R₇ unter Einschließen des Stickstoffatoms jeweils miteinander eine an den Benzolring des Naphthopyrangerüsts gebundene -R₄N-(CH₂)ₖ-X- bzw. -R₃N-(CH₂)ₖ-X- Einheit mit k = 1 oder 2 bilden, wobei X aus O, S, CH₂, C(CH₃)₂, C(C₆H₅)₂, N(CH₃) oder N(C₆H₅) ausgewählt ist und der Rest R₄ bzw. R₃ dann aus Methyl oder Phenyl ausgewählt ist,
wobei an diese -R₄N-(CH₂)ₖ-X- bzw. -R₃N-(CH₂)ₖ-X- Einheit wiederum ein Benzolring annelliert sein kann, oder
die Reste NR₃R₄, R₅ und R₇ zusammen mit dem Benzolring des Naphthopyrangerüsts, an den sie gebunden sind, eine Julolidinyl-Einheit bilden,
B und B' unabhängig voneinander ausgewählt sind aus un-, mono- oder disubstituiertem Phenyl, Ethinyl, Ethenyl, Naphthyl, Furanyl, Benzofuranyl, Thienyl, Benzothienyl oder Julolidinyl, wobei die Substituenten aus der Gruppe α sowie Ethenyl, 4-Ethenylphenyl, Ethinyl oder 4-Ethinylphenyl ausgewählt sind, wobei die Substituenten aus der Gruppe α sowie die vorstehenden Substituenten, soweit möglich, wiederum jeweils unabhängig voneinander zwei oder drei Substituenten aus der Gruppe β aufweisen können, oder wobei zwei direkt benachbarte Substituenten eine X-(CH₂)_{q}-Z-Gruppierung darstellen, wobei q = 1, 2 oder 3 ist und X und Z unabhängig voneinander Sauerstoff, Schwefel, NCH₃, NPh, CH₂, C(CH₃)₂ oder C(C₆H₅)₂ sein können, oder
B und B' zusammen ein un-, mono- oder disubstituiertes 9-Spirofluoren darstellen, wobei die Fluoren-Substituenten aus der Gruppe β ausgewählt sind, oder
B und B' zusammen einen gesättigten Kohlenwasserstoff darstellen, der C₃-C₁₂ spiromonocyclisch, C₇-C₁₂ spirobicyclisch oder C₇-C₁₂ spirotricyclisch ist.

2. 3H-Naphtho[2,1-b]-pyrane nach Anspruch 1, wobei der Rest R₂ aus Phenyl, Naphthyl, Pyridyl, Oxazolyl, Oxadiazolyl, Thiazolyl, Thiadiazolyl, lmidazolyl, Pyrazolyl, Triazolyl, Tetrazolyl, Pyrimidinyl, Pyrazinyl, Acetyl, Benzoyl, Cyan, Formyl, Iminomethyl, 1-lmino-aminomethyl, 1-(Aminomethylen)-2-iminoethyl, N-Hydroxyiminomethyl, Methylenamino, Cyanamino, Dicyanmethyl, Carboxymethyl, (C₁-C₆)-Alkoxycarbonyl, Nitro, Ethenyl, 4-Ethenylphenyl, Ethinyl oder 4-Ethinylphenyl, die jeweils, soweit möglich, ein, zwei, drei oder vier Substituenten aus der Gruppe β aufweisen können, ausgewählt ist oder der Rest R₂ für eine -Y-Naphthyl-Gruppe steht, wobei Y dabei ein Linker zwischen dem Naphthylrest und der Naphthopyraneinheit ist, ausgewählt aus einer Einfachbindung oder aus CH₂, Ethandiyl, Ethendiyl, Ethindiyl, lminomethyl, Phenylen, Biphenyldiyl, Ferrocendiyl, Pyridindiyl, Pyrimidindiyl, Pyrazindiyl oder (Bipyrimidinyl)diyi, und wobei der Naphthylrest einen, zwei, drei oder vier Substituenten aufweisen kann, ausgewählt aus der Gruppe α oder einer -NR₃R₄-Gruppe, wie vorstehend definiert, wobei einer der Naphthylsubstituenten zusammen mit dem Rest R₄ der an den Naphthylrest gebundenen -NR₃R₄ Gruppe eine an den aromatischen Ring des Naphthylrests gebundene -R₄N-(CH₂)ₖ-X-Gruppe mit k = 1 oder 2 bilden kann, wobei der Rest R₄ dann Wasserstoff, einen geradkettigen oder verzweigtkettigen (C₁-C₆)-Alkylrest oder Phenyl darstellt und X Sauerstoff, Schwefel, CH₂, C(CH₃)₂, C(C₆H₅)₂, NCH₃ oder NPh darstellt, und wobei an diese -R₄N-(CH₂)ₖ-X-Gruppe wiederum ein Benzoring annelliert sein kann; oder wobei zwei der Naphthylsubstituenten einen an den Naphthylrest der -Y-Naphthyl-Gruppe annellierten Pyranring bilden, der wiederum mit B und B', wie vorstehend definiert, substituiert sein kann, so daß über den Linker Y gebunden ein zweites photochromes Naphthopyran-System vorliegt;

3. 3H-Naphtho[2,1-b]-pyrane nach Anspruch 1 oder 2, wobei der Rest R₂ für Phenyl, 4-Methoxyphenyl, 4-Nitrophenyl, 4-(2-Pyrimidinyl)-phenyl, 2-Pyridyl, 3-Pyridyl, 4-Pyridyl, 2-Pyrimidinyl. 2-Pyrazinyl, 5-Methyl-2-oxadiazolyl, 2,4-Dimethyl-1-pyrazolyl, 4,5-Dimethyl-2-imidazolyl, 1-Hex-1-inyl, Phenylethinyl oder 4-Pyridylethinyl steht oder der Rest R₂ einen Linker zwischen den 6-Substituenten zweier wie vorstehend definierter 3H-Naphtho[2,1-b]pyrane darstellt, wobei der Linker aus einer direkten Verbindung, Ethendiyl, Ethindiyl, 1,4-Phenylen oder 5,5'-Bipyrimidinyl-2,2'-diyl ausgewählt ist.

4. 3H-Naphtho[2,1-b]-pyrane nach Anspruch 1, 2 oder 3, wobei die Reste R₃ und R₄ gemäß der vorstehenden Formel (1) unabhängig voneinander aus einem unsubstituierten, mono- oder disubstituierten Phenyl-, Phenoxy-, Benzyl-, Benzyloxy-, Naphthyl- oder Naphthoxyrest ausgewählt sind oder zusammen mit dem Stickstoffatom einen wie vorstehend definierten 3- bis 10-gliedrigen Stickstoffheterocyclus bilden.

5. 3H-Naphtho[2,1-b]-pyrane nach Anspruch 4, wobei der Stickstoffheterocyclus eine Morpholingruppe, eine Thiomorpholingruppe, eine Piperidingruppe, eine Azacycloheptangruppe, eine Azacyclooctangruppe, eine 1,4-Diaza-1-methyl-cycloheptangruppe, eine Piperazingruppe, eine (N'-(C₁-C₆-Alkyl)piperazingruppe, eine Pyrrolidingruppe, eine Imidazolidingruppe, eine Pyrazolidingruppe, eine Aziridingruppe, eine Azetidingruppe, eine Indolingruppe, eine Carbazolgruppe, eine Phenothiazingruppe, eine Phenazingruppe, eine Phenoxazingruppe, eine Tetrahydrochinolingruppe oder eine Tetrahydroisochinolingruppe ist.

6. 3H-Naphtho[2,1-b]-pyrane nach Anspruch 4 oder 5, wobei die -NR₃R₄ Gruppe in der vorstehenden Formel (I) in ihrer Gesamtheit für Diphenylamino, Dianisylamino, Morpholinyl, Thiomorpholinyl, 3,5-Dimethylthiomorpholinyl, Piperidinyl, Azacycloheptyl, Azacyclooctyl, 1,4-Diaza-1-methyl-cycloheptyl, Piperazinyl, Pyrrolidinyl oder 1,2,3,4-Tetrahydroisochinolinyl steht.

7. 3H-Naphtho[2,1-b]-pyrane nach einem der Ansprüche 1 bis 6, wobei mindestens einer der Reste B und B' aus einem in para-Stellung mit einer, wie vorstehend definierten -NR₃R₄ Gruppe substituierten Phenylrest ausgewählt ist.

8. Verwendung der photochromen 3H-Naphtho[2,1-b]-pyrane nach einem der Ansprüche 1 bis 7 in Kunststoffmaterialien.

9. Verwendung nach Anspruch 8, wobei das Kunststoffmaterial eine ophthalmische Linse ist.

## Claims

1. Photochromic 3H-naphtho[2,1-b]-pyrans having the general formula (I): 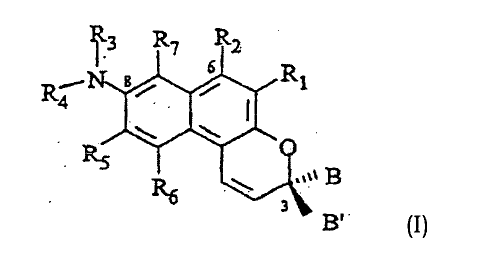 in which
the radicals R₁, R₅, R₆ and R₇, independently of each other, represent hydrogen or a substituent, selected from the group α, comprising fluorine, chlorine, bromine, hydroxy, silyloxy, amino, a straight-chain or branched-chain (C₁-C₆) alkyl radical, a (C₃-C₇) cycloalkyl radical, a straight-chain or branched-chain (C₁-C₆) alkoxy radical, morpholinyl, thiomorpholinyl, piperidinyl, piperazinyl, pyrrolidinyl, imidazolinyl, pyrazolidinyl, 1,2,3,4-tetrahydroquinolinyl, 1,2,3,4-tetrahydroisoquinolinyl, perhydroazepinyl, 4-methyl-perhydro-1,4-diazepinyl, perhydroazocinyl, phenyl, phenoxy, benzyl, benzyloxy, naphthyl, naphthoxy, phenanthryl or pyridyl, which can be substituted respectively with one, two or three substituents, independently of each other, selected from the group β, comprising a straight-chain or branched-chain (C₁-C₆) alkyl radical, a (C₃-C₇) cycloalkyl radical, a straight-chain or branched-chain (C₁-C₆) alkoxy radical, hydroxy, tert.-butyldiphenylsilyloxy, amino, di(C₁-C₆) alkylamino, nitro, cyano, benzyl, 4-methoxybenzyl, 4-nitrobenzyl, phenyl, 4-methoxyphenyl, 4-dimethylaminophenyl, pyridyl and pyrimidinyl;
the radical R₂ is selected from phenyl, naphthyl, phenanthryl, pyridyl, quinolinyl, isoquinolinyl, thienyl, benzothienyl, dibenzothienyl, furanyl, benzofuranyl, dibenzofuranyl, carbazolyl, phenothiazinyl, phenoxazinyl, oxazolyl, benzoxazolyl, oxadiazolyl, thiazolyl, benzothiazolyl, thiadiazolyl, imidazolyl, pyrazolyl, triazolyl, tetrazolyl, pyrimidinyl, pyrazinyl, acetyl, benzoyl, cyano, formyl, iminomethyl, 1-imino-aminomethyl, N-hydroxyiminomethyl, methyleneamino, cyanoamino, cyanomethyl, dicyanomethyl, carboxy, carboxymethyl, (C₁-C₆) acyloxy, (C₁-C₆) alkoxycarbonyl, (C₁-C₆) alkoxycarbonyl-methyl, phenoxycarbonyl, benzyloxycarbonyl, nitro, diazophenyl, aminocarbonyl, ethenyl, 4-ethenylphenyl, ethinyl or 4-ethinylphenyl or with formation of a cationic structure made of N-(C₁-C₆) alkylpyridinio, 1-pyridinio, N-(C₁-C₆) alkylquinolinio, 1-quinolinio, N-(C₁-C₆) alkylisoquinolinio, 2-isoquinolinio, iminiomethyl or 1-iminioaminomethyl, the corresponding counterion being selected from chloride, bromide, sulphate, hydrogen sulphate, tetrafluoroborate, hexafluorophosphate, mesylate, tosylate or triflate, the above substituents being able to have, as far as possible, respectively one, two, three or four substituents from the group β,
or the radical R₂ stands for a Y-naphthyl group, Y being a linker between the naphthyl radical and the naphthopyran unit, selected from a single bond or from CH₂, ethanediyl, ethenediyl, ethinediyl, iminomethyl, phenylene, biphenyldiyl, ferrocenediyl, pyridinediyl, pyrimidinediyl, pyrazinediyl or (bipyrimidinyl)diyl and the naphthyl radical being able to have one, two, three or four substituents, selected from the group α or an -NR₃R₄ group as subsequently defined, one of the naphthyl substituents together with the radical R₄ of the -NR₃R₄ group which is bonded to the naphthyl radical being able to form an -R₄N-(CH₂)ₖ-X group with k = 1 or 2 which is bonded to the aromatic ring of the naphthyl radical, the radical R₄ then representing hydrogen, a straight-chain or branched-chain (C₁-C₆) alkyl radical or phenyl, and X representing oxygen, sulphur, CH₂, C(CH₃)₂, C(C₆H₅)₂, NCH₃ or NPh, and in turn a benzo ring being able to be anellated on this -R₄N-(CH₂)ₖ-X group; or two of the naphthyl substituents forming a pyran ring which is anellated on the naphthyl radical of the -Y-naphthyl group, which ring in turn can be substituted with B and B' as defined subsequently, so that a second photochromic naphthopyran system is present bonded via the linker Y,
the radicals R₃ and R₄, independently of each other, are selected from hydrogen, a straight-chain or branched-chain (C₁-C₆) alkyl radical, a (C₃-C₇) cycloalkyl radical, a (C₁-C₆) alkoxy radical, phenyl or benzyl, the two latter being able to have one or more substituents from the group β, or
the radicals R₃ and R₄ together with the nitrogen atom form an azaadamantyl group or a 3- to 10-membered nitrogen heterocycle, which can be unsubstituted or substituted with a straight-chain or branched-chain (C₁-C₆) alkyl radical, the nitrogen heterocycle being able to contain one or more heteroatoms from the group O, S or NR⁸, the radical R⁸ being selected from hydrogen or the group β and the nitrogen heterocycle being able to be anellated with one or two benzene rings, or
the radicals R₄ and R₅ or R₃ and R₇, with inclusion of the nitrogen atom, form respectively with each other an -R₄N-(CH₂)ₖ-X- or - R₃N-(CH₂)ₖ-X unit with k = 1 or 2 which is bonded to the benzene ring of the naphthopyran framework, X being selected from O, S, CH₂, C(CH₃)₂, C(C₆H₅)₂, N(CH₃) or N(C₆H₅), and the radical R₄ or R₃ then being selected from methyl or phenyl, in turn a benzene ring being able to be anellated on this -R₄N-(CH₂)ₖ-X- or -R₃N-(CH₂)ₖ-X unit, or
the radicals NR₃R₄, R₅ and R₇ together with the benzene ring of the naphthopyran framework, to which they are bonded, form a julolidinyl unit,
B and B', independently of each other, are selected from un-, mono- or disubstituted phenyl, ethinyl, ethenyl, naphthyl, furanyl, benzofuranyl, thienyl, benzothienyl or julolidinyl, the substituents being selected from the group α and also ethenyl 4-ethenylphenyl, ethinyl or 4-ethinylphenyl, the substituents from the group α and also the above substituents being able to have, as far as possible, in turn respectively independently of each other, two or three substituents from the group β, or two directly adjacent substituents representing an X-(CH₂)_{q}-Z grouping, q being equal to 1, 2 or 3 and X and Z, independently of each other, being able to be oxygen, sulphur, NCH₃, NPh, CH₂, C(CH₃)₂ or C(C₆H₅)₂, or
B and B' together represent an un-, mono- or disubstituted 9-spirofluorene, the fluorine substituents being selected from the group β, or
B and B' together represent an saturated hydrocarbon which is C₃-C₁₂ spiromonocyclic, C₇-C₁₂ spirobicyclic or C₇-C₁₂ spirotricyclic.

2. 3H-naphtho[2,1-b]-pyrans according to claim 1, the radical R₂ being selected from phenyl, naphthyl, pyridyl, oxazolyl, oxadiazolyl, thiazolyl, thiadiazolyl, imidazolyl, pyrazolyl, triazolyl, tetrazolyl, pyrimidinyl, pyrazinyl, acetyl, benzoyl, cyano, formyl, iminomethyl, 1-imino-aminomethyl, 1-(aminomethylene)-2-iminoethyl, N-hydroxyiminomethyl, methyleneamino, cyanoamino, dicyanomethyl, carboxymethyl, (C₁-C₆) alkoxycarbonyl, nitro, ethenyl, 4-ethenylphenyl, ethinyl or 4-ethinylphenyl which respectively, as far as possible, can have one, two, three or four substituents from the group β, or the radical R₂ standing for a -Y-naphthyl group, Y thereby being a linker between the naphthyl radical and the naphthopyran unit, selected from a single bond or from CH₂, ethanediyl, ethenediyl, ethinediyl, iminomethyl, phenylene, biphenyldiyl, ferrocenediyl, pyridinediyl, pyrimidinediyl, pyrazinediyl or (bipyrimidinyl)diyl, and the naphthyl radical being able to have one, two, three or four substituents, selected from the group α or an -NR₃R₄ group, as defined above, one of the naphthyl substituents, together with the radical R₄ of the -NR₃R₄ group which is bonded to the naphthyl radical being able to form an -R₄N-(CH₂)ₖ-X group with k = 1 or 2 which is bonded to the aromatic ring of the naphthyl radical, the radical R₄ then representing hydrogen, a straight-chain or branched-chain (C₁-C₆) alkyl radical or phenyl, and X representing oxygen, sulphur, CH₂, C(CH₃)₂, C(C₆H₅)₂, NCH₃ or NPh, and in turn a benzo ring being able to be anellated on this -R₄N-(CH₂)ₖ-X group; or two of the naphthyl substituents forming a pyran ring which is anellated on the naphthyl radical of the -Y-naphthyl group and which in turn can be substituted with B and B', as defined above, so that a second photochromic naphthopyran system is present bonded via the linker Y.

3. 3H-naphtho[2,1-b]-pyrans according to claim 1 or 2, the radical R₂ standing for phenyl, 4-methoxyphenyl, 4-nitrophenyl, 4-(2-pyrimidinyl)-phenyl, 2-pyridyl, 3-pyridyl, 4-pyridyl, 2-pyrimidinyl, 2-pyrazinyl, 5-methyl-2-oxadiazolyl, 2,4-dimethyl-1-pyrazolyl, 4,5-dimethyl-2-imidazolyl, 1-hex-1-inyl, phenylethinyl or 4-pyridylethinyl or the radical R₂ representing a linker between the 6-substituents of two 3H-naphtho[2,1-b]-pyrans as defined above, the linker being selected from a direct compound, ethenediyl, ethinediyl, 1,4-phenylene or 5,5'-bipyrimidinyl-2,2'-diyl.

4. 3H-naphtho[2,1-b]-pyrans according to claim 1, 2 or 3, the radicals R₃ and R₄ according to the above formula (1) being selected, independently of each other, from an unsubstituted, monosubstituted or disubstituted phenyl, phenoxy, benzyl, benzyloxy, naphthyl or naphthoxy radical, or forming together with the nitrogen atom a 3- to 10-membered nitrogen heterocycle as defined above.

5. 3H-naphtho[2,1-b]-pyrans according to claim 4, the nitrogen heterocycle being a morpholine group, a thiomorpholine group, a piperidine group, an azacycloheptane group, an azacyclooctane group, a 1,4-diaza-1-methyl-cycloheptane group, a piperazine group, an (N'-(C₁-C₆-alkyl) piperazine group, a pyrrolidine group, an imidazolidine group, a pyrazolidine group, an aziridine group, an azetidine group, an indoline group, a carbazole group, a phenothiazine group, a phenazine group, a phenoxazine group, a tetrahydroquinoline group or a tetrahydroisoquinoline group.

6. 3H-naphtho[2,1-b]-pyrans according to claim 4 or 5, the NR₃R₄ group in the above formula (I) standing in total for diphenylamino, dianisylamino, morpholinyl, thiomorpholinyl, 3,5-dimethylthiomorpholinyl, piperidinyl, azacycloheptyl, azacyclooctyl, 1,4-diaza-1-methyl-cycloheptyl, piperazinyl, pyrrolidinyl or 1,2,3,4-tetrahydroisoquinolinyl.

7. 3H-naphtho[2,1-b]-pyrans according to one of the claims 1 to 6, at least one of the radicals B and B' being selected from a phenyl radical which is substituted in paraposition with an -NR₃R₄ group as defined above.

8. Use of the photochromic 3H-naphtho[2,1-b]-pyrans according to one of the claims 1 to 7 in plastic materials.

9. Use according to claim 8, the plastic material being an ophthalmic lens.

## Revendications

1. 3N-Naphto[2,1-b]-pyranne photochrome de formule générale (I) : 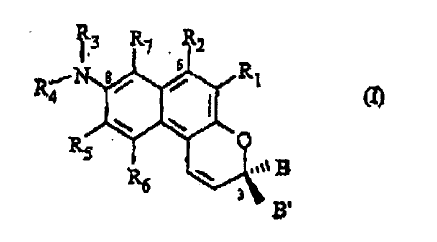 dans laquelle
les radicaux R₁, R₅, R₆ et R₇ désignent, indépendamment les uns des autres, l'hydrogène ou un substituant, sélectionné parmi le groupe α, se composant des radicaux fluor, chlore, brome, hydroxy, silyloxy, amine, d'un radical alkyle en C₁-C₅ linéaire ou ramifié, d'un radical cycloalkyle en C₃-C₇, d'un radical alkoxy en C₁-C₆ linéaire ou ramifié, de radicaux morpholinyle, thiomorpholinyle, pipéridinyle, pipérazinyle, pyrrolidinyle, imidazolinyle, pyrazolidinyle, 1,2,3,4-tétrahydroquinoléinyle, 1,2,3,4-tétrahydroisoquinoléinyle, perhydroazépinyle, 4-méthyl-perhydro-1,4-diazépinyle, perhydroazocinyle, phényle, phénoxy, benzyle, benzyloxy, naphtyle, naphtoxy, phénanthryle ou pyridyle, qui peuvent être substitués, à chaque fois, par un, deux ou trois substituants, sélectionnés indépendamment les uns des autres parmi le groupe β, se composant d'un radical alkyle en C₁-C₆, linéaire ou ramifié, d'un radical cycloalkyle en C₃-C₇, d'un radical alkoxy en C₁-C₆ linéaire ou ramifié, des radicaux hydroxy, tert.-butyldiphénylsilyloxy, amino, di(C₁-C₆)alkylamino, nitro, cyano, benzyle, 4-méthoxybenzyle, 4-nitrobenzyle, phényle, 4-méthoxyphényle, 4-diméthylaminophényle, pyridyle et pyrimidinyle;
le radical R₂ est sélectionné parmi les radicaux phényle, naphtyle, phénanthryle, pyridyle, quinoléinyle, isoquinoléinyle, thiényle, benzothiényle, dibenzothiényle, furanyle, benzofuranyle, dibenzofuranyle, carbazolyle, phénothiazinyle, phénoxazinyle, oxazolyle, benzoxazolyle, oxadiazolyle, thiazolyle, benzothiazolyle, thiadiazolyle, imidazolyle, pyrazolyle, triazolyle, tétrazolyle, pyrimidinyle, pyrazinyle, acétyle, benzoyle, cyano, formyle, iminométhyle, 1-iminoaminométhyle, N-hydroxyiminométhyle, méthylèneamino, cyanamino, cyanométhyle, dicyanométhyle, carboxy, carboxyméthyle, acyloxy en C₁-C₆, alkoxycarbonyle en C₁-C₆, alkoxycarbonylméthyle en C₁-C₆, phénoxycarbonyle, benzyloxycarbonyle, nitro, diazophényle, aminocarbonyle, éthényle, 4-éthénylphényle, éthynyle ou 4-éthynylephényle ou sous formation d'une structure cationique faite à partir de N-(C₁-C₆)-alkylpyridinio, 1-pyridinio, N-(C₁-C₈)-alkylquinoléinio, 1-quinoléinio, N-(C₁-C₆)-alkylisoquinoléinio, 2-isoquinoléionio, iminiométhyle ou 1-iminioaminométhyle, le contre-ion correspondant étant alors sélectionné parmi les ions chlorure, bromure, sulfate, hydrogénosulfate, tétrafluoroborate, hexafluorophosphate, mésylate, tosylate ou triflate, les substituants suscités pouvant présenter, dans la mesure du possible, à chaque fois, un, deux trois ou quatre substituants provenant du groupe β, ou le radical R₂ représente un groupement -Y-naphtyle, Y étant, en l'occurrence, un agent de liaison entre le radical napthyle et l'unité de naphtopyranne, sélectionné parmi une liaison simple ou parmi CH₂, éthanediyle, éthenedyle, éthynediyle, iminométhyle, phénylène, biphényldiyle, ferrocènediyle, pyridinediyle, pyrimidinediyle, pyrazinediyle ou (bipyrimidinyl)diyle, et le radical naphtyle pouvant présenter un, deux, trois ou quatre substituants, sélectionnés parmi le groupe α ou un groupe -NR₃R₄-, comme défini ci-après, un des substituants naphtyle, conjointement au radical R₄ du groupement -NR₃R₄ lié au cycle aromatique du radical naphtyle, pouvant former un groupement -R₄N-(CH₂)ₖ-X-, lié au cycle aromatique du radical naphtyle, avec k = 1 ou 2, le radical R₄ représentant alors l'hydrogène, un radical alkyle en C₁-C₆ ou un radical phényle, linéaire ou ramifié, et X représentant l'oxygène, le soufre, CH₂, C(CH₃)₂, C(C₆H₅)₂, NCH₃ ou NPh, et un cycle benzène pouvant à nouveau être fusionné à ce groupement R₄N-(CH₂)ₖ-X-,; ou deux des substituants napthyle formant un cycle pyranne fusionné au radical naphtyle du groupement -Y-naphtyle, qui peut à nouveau être substitué à l'aide de B et de B', comme défini ci-après, de telle sorte qu'un second système naphtopyranne photochrome soit présent par l'intermédiaire d'une liaison à l'aide de l'agent de liaison Y;
les radicaux R₃ et R₄ sont sélectionnés, indépendamment l'un de l'autre, parmi l'hydrogène, un radical alkyle en C₁-C₆ linéaire ou ramifié, un radical cycloalkyle en C₃-C₇, un radical alkoxy en C₁-C₆, un radical phényle ou benzyle, les deux derniers radicaux pouvant présenter un ou plusieurs substituants provenant du groupe β ou
les radicaux R₃ et R₄, conjointement à l'atome d'azote, forment un groupement aza-adamantyle ou un cycle hétérocyclique d'azote de 3 à 10 membres, qui peut être non substitué ou substitué par un radical alkyle en C₁-C₆ linéaire ou ramifié, le cycle hétérocyclique d'azote pouvant contenir un ou plusieurs hétéroatomes provenant du groupe O, S ou NR⁸, le radical R⁸ étant sélectionné parmi l'hydrogène ou le groupe β, et le cycle hétérocyclique d'azote pouvant être fusionné avec un ou deux cycles benzéniques, ou
les radicaux R₄ et R₅ ou R₃ et R₇ formant, sous inclusion de l'atome d'azote, à chaque fois, une unité -R₄N-(CH₂)ₖ-X- ou -R₃N-(CH₂)ₖ-X-, liée au cycle benzénique du squelette naphtopyranne avec k =1 ou 2, X étant sélectionné parmi O, S, CH₂, C(CH₃)₂, C(C₆H₅)₂, N(CH₃) ou N(C₆H₅) et le radical R₄ ou R₃ étant sélectionné alors parmi les radicaux méthyle ou phényle, un cycle benzénique pouvant à nouveau être fusionné à cette unité -R₄N-(CH₂)ₖ-X- ou -R₃N-(CH₂)ₖ-X-, ou
les radicaux NR₃R₄, R₅ et R₇, formant, conjointement avec le cycle benzénique du squelette naphtopyranne, auxquels ils sont attachés, une unité julolidinyle,
B et B' sont sélectionnés indépendamment l'un de l'autre parmi des radicaux non substitués, mono-substitués ou di-substitués phényle, éthynyle, éthényle, naphtyle, furanyle, benzofuranyle, thiényle, benzothiényle ou julolidinyle, les substituants étant sélectionnés parmi le groupe α ainsi que les radicaux éthényle, 4-éthénylphényle, éthynyle ou 4-éthynylphényle, les substituants provenant du groupe α ainsi que les substituants suscités pouvant présenter, indépendamment les uns des autres, dans la mesure du possible, à chaque fois, à nouveau, deux ou trois substituants provenant du groupe β ou deux substituants directement voisins représentant un groupement X-(CH₂)_{q}-Z-, q étant égal à 1, 2 ou 3 et X et Z pouvant être, indépendamment l'un de l'autre, l'oxygène, le soufre, NCH₃, NPh, CH₂, C(CH₃)₂ ou C(C₆H₅)₂, ou
B et B' représentent, conjointement, un 9-spirofluorène non substitué, mono-substitué ou di-substitué, les substituants du fluorène étant sélectionnés parmi le groupe β ou
B et B', conjointement, représentent un hydrocarbure saturé, qui est C₃-C₁₂-spiro-monocyclique, C₇-C₁₂-spiro-bicyclique ou C₇-C₁₂-spiro-tricyclique,

2. 3H-Naphto[2,1-b]-pyranne selon la revendication 1, le radical R₂ étant sélectionné parmi les radicaux phényle, naphtyle, pyridyle, oxazolyle, oxadiazolyle, thiazolyle, thiadiazolyle, imidazolyle, pyrazolyle, triazolyle, tétrazolyle, pyrimidinyle, pyrazinyle, acétyle, benzoyle, cyano, formyle, iminométhyle, 1-imino-aminométhyle, 1-(aminométhylène)-2-iminoéthyle, N-hydroxyiminométhyle, méthylenamino, cyanamino, dicyanométhyle, carboxyméthyle, alkoxycarbonyle en C₁-C₆, nitro, éthényle, 4-éthénylphényle, éthynyle ou 4-éthynylphényle, qui peuvent présenter, à chaque fois, dans la mesure du possible, un, deux, trois ou quatre substituants provenant du groupe β ou le radical R₂ représentant un groupement -Y-naphtyle, Y étant, en l'occurrence, un agent de liaison entre le radical naphtyle et l'unité naphtopyranne, sélectionné parmi une liaison simple ou CH₂, éthanediyle, éthénediyle, éthynediyle, iminométhyle, phénylène, biphényldiyle, ferrocénediyle, pyridinediyle, pyrimidinediyle, pyrazinediyle ou (bipyrimidinyl)diyle, et le radical naphtyle pouvant présenter un, deux, trois ou quatre substituants, sélectionné parmi le groupe α ou un groupement -NR₃R₄-, comme défini ci-devant, un des substituants naphtyle, conjointement au radical R₄ du groupe -NR₃R₄ lié au cycle aromatique du radical naphtyle, pouvant former un groupement -R₄N-(CH₂)ₖ-X-, lié au cycle aromatique du radical naphtyle avec k = 1 ou 2, le radical R₄ représentant alors l'hydrogène, un radical alkyle en C₁-C₆ ou un phényle linéaire ou ramifié et X représentant l'oxygène, le soufre, CH₂, C(CH₃)₂, C(C₅H₅)₂, NCH₃ ou NPh, et un cycle benzénique pouvant à nouveau être fusionné à ce groupement -R₄N-(CH₂)ₖ-X-; ou deux des substituants de naphtyle formant un cycle de pyranne fusionné au radical naphtyle du groupment -Y-naphtyle, qui peut à nouveau, être substitué à l'aide de B et B', comme défini précédemment, de telle sorte qu'un second système napthopyranne photochrome soit présent, lié par l'intermédiaire de l'agent de liaison Y.

3. 3H-Naphto[2,1-b)-pyranne selon la revendication 1 ou 2, le radical R₂ représentant les radicaux phényle, 4-méthoxyphényle, 4-nitrophényle, 4-(2-pyrimidiny)-phényle, 2-pyridyle, 3-pyridyle, 4-pyridyle, 2-pyrimidinyle, 2-pyrazinyle, 5-méthyl-2-oxadiazolyle, 2,4-diméthyl-1-pyrazolyle, 4,5-diméthyl-2-imidazolyle, 1-hex-1-ynyle, phényléthynyle ou 4-pyridyléthynyle ou le radical R₂ représente un agent de liaison entre les substituants en position 6 de deux 3H-naphto[2,1-b]pyrannes définis comme ci-devant, l'agent de liaison étant sélectionné parmi une liaison directe éthénediyle, éthynediyle, 1,4-phénylène ou 5,5'-bipyrimidinyl-2,2'-diyle.

4. 3H-Naphto[2,1-b]-pyranne selon la revendication 1, 2 ou 3, les radicaux R₃ et R₄ étant sélectionnés, conformément à la formule précédente (I), indépendamment l'un de l'autre, parmi un radical phényle, phénoxy, benzyle, benzyloxy, naphtyle ou naphtoxy ou formant, conjointement à l'atome d'azote, un cycle hétérocyclique d'azote, comme défini ci-devant, ayant de 3 à 10 membres.

5. 3H-Naphto[2,1-b]-pyranne selon la revendication 4, le cycle hétérocyclique d'azote étant un groupement morpholine, un groupement thiomorpholine, un groupement pipéridine, un groupement azacycloheptène, un groupement azacyclooctane, un groupement 1,4-diaza-1-méthyl-cycloheptane, un groupement pipérazine, un groupement (N'-(C₁-C₆-alkyl)pipérazine, un groupement pyrrolidine, un groupement imidazolidine, un groupement pyrazolidine, un groupement aziridine, un groupement azétidine, un groupement indoline, un groupement carbazole, un groupement phénothiodiazine, un groupement phénazine, un groupement phénoxazine, un groupement tétrahydroquinoléinyle ou un groupement tétrahydroisoquinoléinyle.

6. 3H-Naphto[2,1-b]-pyranne selon la revendication 4 ou 5, le groupement -NR₃R₄ représentant dans la formule précédente (I) dans son ensemble un radical diphénylamino, dianisylamino, morpholinyle, thiomorpholinyle, 3,5-diméthylthiomorpholinyle, pipéridinyle, azacycloheptyle, azacyclooctyle, 1,4-diaza-1-méthyl-cycloheptyle, pipérazinyle, pyrrolidinyle ou 1,2,3,4-tétrahydroisoquinoléinyle.

7. 3H-Naphto[2,1-b]-pyranne selon l'une quelconque des revendications 1 à 6, au moins un des radicaux B et B' étant sélectionné parmi un radical phényle substitué, en position para, à l'aide d'un groupment -NR₃R₄, défini comme précédemment.

8. Utilisation du 3H-naphto[2,1-b]-pyranne photochrome selon l'une quelconque des revendications 1 à 7 dans des matériaux synthétiques.

9. Utilisation selon la revendication 8, le matériau synthétique étant une lentille ophtalmique.
